# EUROPEAN PATENT APPLICATION

(11) **EP 2 980 203 A1**
(43) Date of publication of application: **03.02.2016**
(21) Application number: 14382289.8
(22) Date of filing: 29.07.2014
(51) Int. Cl.: C12M 1/107, C12M 1/00, C12M 1/06, C12P 5/02, B09B 3/00

(54) **Anaerobic digester for the treatment of organic waste**

(71) Applicant: Fomento de Construcciones y Contratas, S.A., 08007 Barcelona (ES)
(72) Inventor: ARRIBAS DE PAZ, Ricardo, 21819 Huelva (ES)
(74) Representative: Carvajal y Urquijo, Isabel

(57) **Abstract**

Anaerobic digester and method for the treatment of organic waste which comprises 3 equally sized spherical chambers (1, 2 and 3) positioned at the same height and joined by interconnecting pipes (4, 5 and 6). Each chamber houses a mixing mechanism consisting of a horizontally rotating propeller with two flat paddles that are symmetrical to the axis of rotation and coplanar with each other and this axis.

Each of the spherical chambers has an outlet on the top for extraction of biogas, an outlet on the bottom for totally draining the chamber, two connection points for the interconnecting pipes (4, 5 and 6) and an outlet for treated digestate on the bottom half of the chamber. Two of the chambers (1 and 2) also have a loading inlet connected to the tubes (15) from the storing tank (16) for treatable waste.

## Description

### Field of the invention

This invention refers to an anaerobic digester for the treatment of organic waste of the type that comprises a number of chambers arranged in series with interconnecting pipes, inlets and outlets for the material to be treated, an outlet for the biogas produced and an internal mechanism for mixing and/or blending the material inside the chambers.

The digester can be used, for example, to treat the organic part of municipal solid waste (MSW), waste from cattle and/or agricultural farming, organic marine material such as algae, waste wood such as pruned branches, etc. It is a single-stage device that can be used in continuous, semi-continuous or batch mode.

Anaerobic digestion systems can be classified according to their complexity (single-stage, multistage), the operating temperature (mesophilic or thermophilic), the operating regime of the digester (continuous, semi-continuous or batch) and, lastly, according to the concentration of total solids (TS) (wet or dry fermentation systems).

According to the above, this invention can be classified as a single-stage digester that can be used in continuous, semi-continuous or batch mode. It functions as either a mesophilic or thermophilic digester and allows for both wet and dry fermentation.

### Background to the invention

The increased production of organic waste in recent years, especially municipal solid waste (MSW), has created the need for more efficient waste management to minimise its impact on the environment.

The legislation currently in force in the majority of countries includes the "Reduction of Dumping Biodegradable Waste" as a key strategy for reducing its environment impact. As such, the need arising to treat biodegradable waste, especially municipal solid waste (MSW) due to its high percentage of organic material, using biological techniques including, among others, anaerobic digestion or biomethanisation, through which biogas can also be produced.

Biogas represents approximately 5% of the energy generated from biomass in Europe. One of its key uses, among others, is to produce electricity that is then fed into the grid. Heat recovery and cogeneration are also possible. Biogas is often used exclusively within the plant at which it is produced.

There are many different types of anaerobic digester due to the varied composition of the waste to be treated, the wide choice of operating parameters (residence time, concentration of solids, mixing, recirculation, inoculation, number of stages, temperature, etc.) and, being a process that is still under development, the fact that there is still no single optimal technology.

In general, digesters are made from two or more chambers connected in series with the process occurring progressively between the first and the last chambers, which usually have different characteristics. The material to be treated is loaded into the first chamber and the treated material and the biogas are extracted from the last chamber.

With both single-chamber and multi-chamber digesters, the chambers are usually cylindrical and will rotate around either a vertical or horizontal axis.

Anaerobic digesters with these characteristics are described, for example, in the following patents: ES2209613, ES2249960, US2010044307, NO861276, SI1848675 and PT1841853.

### Description of the invention

The aim of this invention is to create an anaerobic digester for the treatment of organic waste designed to perform, with maximum efficiency, three different functions: the production of biogas; the production of solid waste, which after composting can be used as fertiliser or fuel; and the elimination of the organic waste introduced for treatment.

This digester is of the type described above, comprising a series of chambers, ideally three. These chambers, according to one key feature of the invention, are spherical, all the same size, equidistant from each other and all positioned at the same height.

The advantage of spherical chambers is that this shape minimises deposition and sedimentation at the bottom of the chamber and facilitates maintenance and cleaning tasks. In addition, the spherical form offers an optimal volume-to-surface ratio, allowing for a cost-effective use of material.

Each of the digester chambers is fitted with a continuous and low-speed mixing mechanism, to prevent turbulence inside the chamber. According to another feature of the invention, the mixing mechanism consists of a horizontally rotating propeller that passes through the centre of the chamber. The propeller has two paddles that are symmetrical to the axis of rotation and coplanar with each other and this axis.

According to the preferred configuration, the propeller paddles have a circular-sector profile, a radius that is slightly lower than the internal radius of the module and are positioned opposite each other in line with the axis of rotation.

The pipes that connect the chambers run along the sides of the equilateral triangle formed between the three spherical chambers. The axis of rotation of the propeller in each chamber is on the same line as the bisector of the angles of said triangle. As a result, the propeller paddles, the main component of the mixing mechanism, do not interfere with the points at which the interconnecting pipes join each chamber.

The blending and mixing paddles, thanks to the characteristics described above, ensure an even consistency of the material inside the chambers through continuous mixing. In addition, the shape and size of the paddles prevents material from building up at the bottom of the chamber or in other part of same since the areas that are not affected by the paddles are sufficiently angled so the viscosity of the digestate causes neither deposition nor accumulation.

A fundamental aspect of anaerobic digestion is the creation of a optimal balance between waste treatment and energy production. Both these processes require maximum homogenisation of the waste material. That is to say, all treated material must be broken down to a similar degree.

Uniformity in the "age" of the waste being treated minimizes the longer it remains within the treatment process. However, according to proven research, this is only really effective up to the use of 3 chambers: the improvements gained by adding a fourth chamber are relatively insignificant compared to those achieved by changing from 1 to 3 modules.

This is why, according to the preferred configuration, this digester is equipped with 3 modules positioned at the vertices of a equilateral triangle, with the centres located on a horizontal plane. The chambers are all interconnected and equipped with two outlets located at opposite ends; the top outlet for the evacuation of biogas and the bottom outlet for the evacuation of effluents. Two of the chambers also have inlets for loading the waste to be treated.

With this configuration, in the event of malfunction or if any maintenance is required, each chamber can be isolated without disrupting the process and with an acceptable loss of efficiency.

According to another possible configuration, the chambers are interconnected by pipes that run along the horizontal centre line of the chambers. The cross section of these pipes decreases, at least on their lower half, from their connection point with the chambers to their central section, which is achieved, for example, with a semi-tapered design. This feature prevents material from accumulating in these interconnecting pipes, allowing it to pass from one chamber to the next thanks to a basic difference in pressure which is created each time digestate is extracted or new waste is introduced.

Ideally, the loading inlets found on two of the chambers are located on the lower half of the chamber and are connected to a storage tank for treatable waste that is positioned at a lower height than the loading inlets, so the waste to be treated flows upwards from the tank to the chambers.

The loading inlets, outlets and pipes that connect the chambers are all equipped with shutoff valves or gates. In the interconnecting pipes, the valve or gate is positioned at the centre of the section of these pipes with the smallest cross section.

In order facilitate cleaning and maintenance tasks, the chambers have an access gate on their lower half.

This digester also has a continuous monitoring system through which the transformation processes, production of biogas and characteristics of the output waste can all be controlled. The system includes supervision, control and data-acquisition devices to facilitate real-time feedback and automatic process control. It provides all information generated throughout the production process (supervision, quality control, production control, data storage, etc.) and allows for management and invention in said process.

In addition, the system also has a safety mechanism that limits the pressure of the gas produced by anaerobic fermentation in each chamber.

Lastly, the digester is also equipped with devices for storing recoverable products and sub-products, a gas holder for the biogas and a drain for maintenance and cleaning purposes.

All component of the digester are made from very durable, non-corrodible material that is highly resistant to the temperature, humidity and pressure required for fermentation.

Another aim of the invention is the anaerobic treatment of organic waste using a method of progressive and continuous degradation through at least three equally sized and equidistant spherical modules or chambers, positioned at the same height and connected in series. In each of these modules, the waste is homogenised and treated through continuous mixing. Treatable waste is selectively loaded through one of the loading inlets found on two of the modules, while the treated digestate is selectively extracted from any of the three modules and the biogas is extracted simultaneously from all modules.

In basic terms, the invention has two key processes. The first is the fermentation of waste, which occurs within the digesters. This process begins with the introduction of organic material through the loading tube connected to the first digester and finishes with the extraction of biogas on the one hand, and digestate on the other, through their respective system of pipes. The second process is the continuous monitoring of each of the spherical chambers and, as a result, of the overall functioning of the system. This is achieved by the introduction of supervision, control and data-acquisition devices that allow the entire productive system to be controlled and monitored remotely and in real time. It also allows maintenance and cleaning tasks to be planned and scheduled.

### Brief description of the diagrams

The features and benefits of the invention are better understood with an example of one of the ways in which it can be set up, as shown in the attached diagrams, in which:
- Figure 1 shows a perspective view of one way of setting up the digester.
- Figure 2 is a bird's eye view of the digester, showing a horizontal cross section of the chambers.
- Figure 3 shows a vertical cross section of one of the interconnecting pipes that run between the digester chambers.
- Figure 4 is a cross section of one of the chambers, cut along the IV-IV line in Figure 2.

### Detailed description of one operating mode

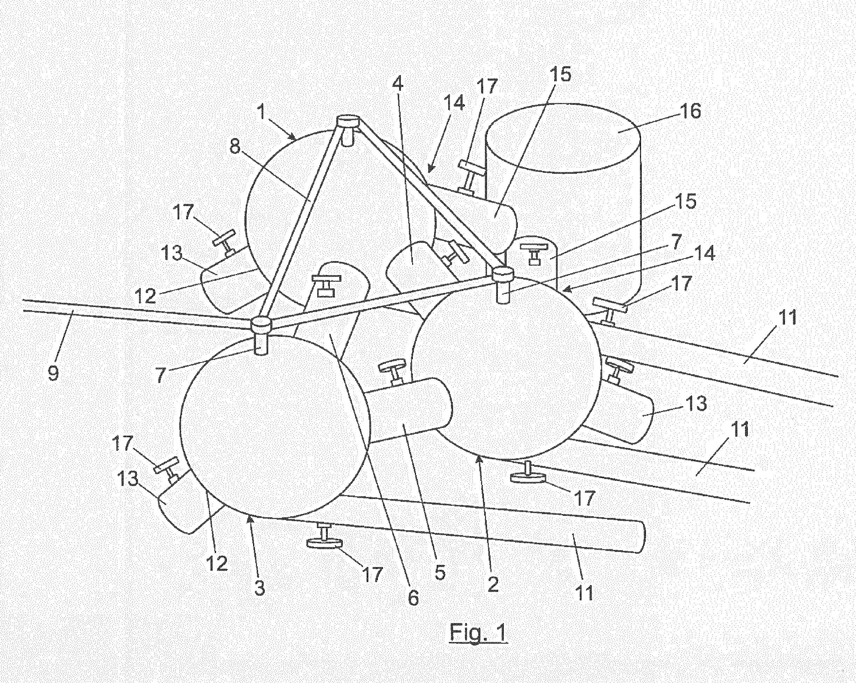

Figure 1 shows a digester configured according to the invention, which has 3 spherical chambers (1, 2 and 3) all with the same radius, positioned at an equal distance apart at the vertices of an equilateral triangle and all at the same height.

Interconnecting pipes run between these 3 chambers (4, 5 and 6) and transport the waste for treatment from one chamber to the next. The axes of these pipes run along the horizontal centre line of the spherical chambers. Each spherical chamber has an outlet at the top (7) for extracting the biogas produced within the chamber. This outlet is connected to a pipe (8) that in turn leads to a general pipe (9) through which the biogas from all three chambers is channelled to a gas holder (not shown).

At the bottom of each chamber (1, 2 and 3) there is an outlet (10) for the extraction of effluents. The pipes (11) connected to this outlet lead to a common waste collector (not shown).

The spherical chambers also have a discharge outlet (12) for solid treated waste, located on the bottom half of the chamber, which is connected to an outlet pipe (13).

Lastly, two of the spherical chambers, those identified as 1 and 2, have a waste loading inlet (14) connected by a system of tubes (15) to a waste storage tank (16). These tubes (15) run on an upward slope from the storage tank to the corresponding chamber, connecting with the waste loading inlet on the lower half of the chamber.

All pipes (4, 5, 6, 11, 13 and 15), including the biogas extraction pipe (8), are fitted with shutoff valves (17).

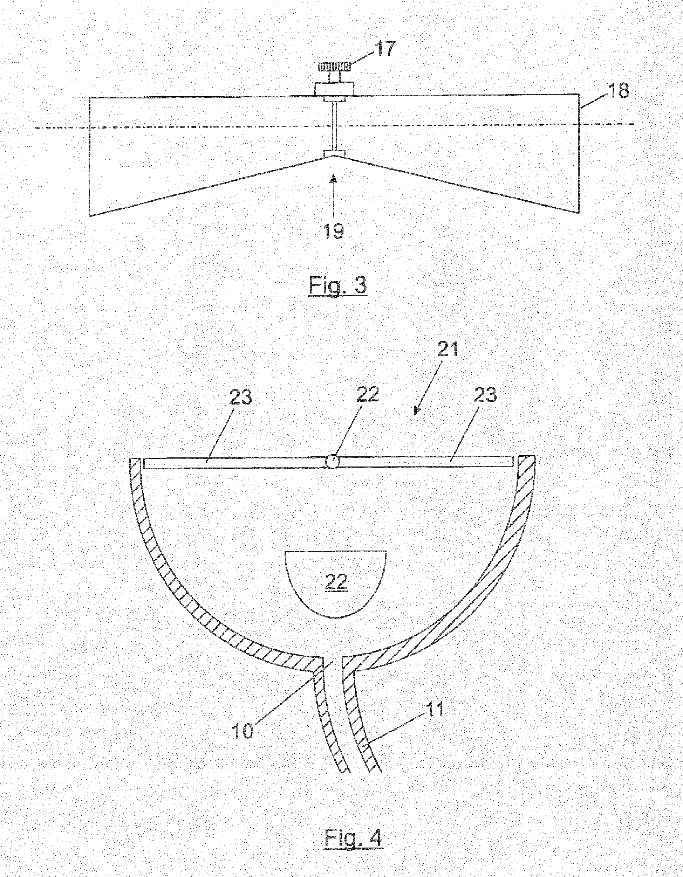

As shown in Figure 3, the pipes (4, 5 and 6) which connect the 3 chambers (1, 2 and 3) are tapered, at least on their lower half, from their connection point with the chambers (18) to their central section (19), where the shutoff valve is located (17).

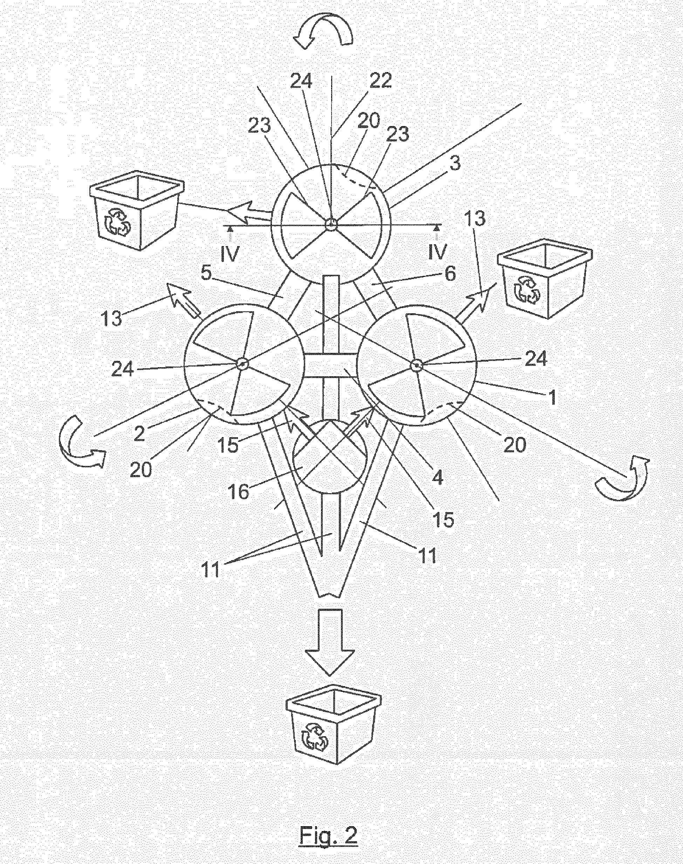

Each spherical chamber has an access gate (20) on the lower half of the chamber, as seen in Figure 2.

Each spherical chamber houses a mixing device, as seen in Figures 2 and 4, consisting of a propeller (21) that rotates on a horizontal axis (22), which passes through the centre of the chamber, and is fitted with 2 flat paddles (23) that are symmetrically mounted in relation to the axis of rotation (22). The two paddles are coplanar with each other and this axis (22). The paddles (23) have a circular-sector profile, and their radius is approximately equal to or slightly lower than the internal radius of the spherical chambers.

As seen in Figure 2, the axis (22) of the propeller in each of the spherical chambers is in line with the bisector of the 60º angles of the equilateral triangle formed at the centre (24) of the 3 spherical chambers. As a result, as the propeller (21) rotates, the paddles (23) do not interfere with the connection points of the interconnecting pipes (4, 5 and 6) or with the access gate (20).

The digester, when configured as described, is loaded or fed regularly, for example on a daily basis, with a relatively small amount of waste compared to the total content of the digester, while an equal volume of digestate is extracted from the digester and, as such, a constant volume is maintained in each of the 3 chambers.

Waste is fed into one of the chambers (1 or 2) that is fitted with loading inlets (14). The last chamber will be in third position depending on how the digester is set up, from left to right or vice versus, according to the individual case, since the interconnecting pipes (4, 5 and 6) are all the same length.

Biogas is, in general, produced continually in each of the spherical chambers due to the constant supply of nutrients received by the methanogens, which are responsible for generating this gas.

The digester as described has an anaerobic digestion system comprised of three equally sized spherical units, arranged in series, with a semi-continuous operating regime for the production of biogas, mainly methane.

When feeding waste into one of the two chambers, one of them can be bypassed and isolated in the event of malfunction or maintenance.

The equilateral triangular layout and that fact that all 3 chambers and interconnecting pipes are alike allows for standardised construction of the digester.

New material from the storage tank (16), Figure 1, is introduced into the system through tubes (15) that run on an upward slope, for example 45º, to the corresponding chamber and are connected on the lower half of the chamber. The upward flow of waste into the digester enhances the anaerobic effect in the tubes, so that the waste moves through them in order to equalise the pressure when the corresponding shutoff valve (17) is opened. The possibility of feeding new waste into the digester through just one of the chambers (1 or 2) allows us to choose the chamber in which the process will begin, in order to isolate either one of the 2 remaining chambers for maintenance or repair work, without having to stop the process.

The pipes (13) for extracting the treated material run on a downward slope, for example 45º, allowing for the digestate to easily flow down them when the corresponding valve (17) is opened.

The propeller (21) in each chamber mixes the material continuously and at a low speed to prevent turbulence inside the chambers.

The configuration and size of the paddles (23) on the propeller (21), as seen in Figures 2 and 4, encourages maximum contact between the fresh substrate and the bacteria, preventing both the formation of a floating layer of foam and sedimentation (deposits at the bottom of the chamber). It also prevents the creation of any dead spaces which reduce the effective volume, the elimination of preferred paths and it stops thermal stratification by maintaining a uniform temperature throughout the digester, which in turn increases the overall speed of the process.

The digester includes a continuous monitoring system through which the transformation processes, production of biogas and characteristics of the output waste can all be controlled. The system includes supervision, control and data-acquisition devices to facilitate real-time feedback and automatic process control. It provides all information generated throughout the production process (supervision, quality control, production control, data storage, etc.) and allows for management and invention in said process. In addition, the system also has a safety mechanism that limits the pressure of the gas produced by anaerobic fermentation in each chamber.

With this digester, waste is treated via progressive and continuous degradation, as described above.

That is to say, that with this method, the treatment of waste is continuous and all stages in the process are performed in each of the spherical chambers, although the proportion of the different processes is not the same in each chamber.

## Claims

1. An anaerobic digester for the treatment of organic waste comprising a series of chambers or modules, connected in series by interconnecting pipes and fitted with inlets for loading the material to be treated, a discharge outlet for treated material, an outlet for biogas, a drain and an internal mixing mechanism, **characterised by** the fact that:
- The chambers are spherical, of equal dimensions and all positioned at the same height;
- The mixing mechanism in each chamber consists of a horizontally rotating propeller, aligned with the centre line of the sphere and fitted with two flat paddles that are symmetrical to the axis of rotation and coplanar with each other and this axis;
- Each chamber has two outlets, one at each opposite end, the top one for the extraction of biogas and the bottom one for fully draining the chamber;
- Two of the chambers also have an inlet for loading new waste; AND,
- Each chamber has 2 connection points for the pipes running to and from its neighbouring chambers and an outlet for the extraction of treated digestate, located on the lower half of the chamber.

2. Digester according to Claim 1, **characterised by** the fact that the axes of the pipes that connect the chambers are aligned with the horizontal centre line of the chambers.

3. Digester according to Claim 2, **characterised by** the fact that the interconnecting pipes that run between the chambers have a decreasing cross section, at least in their lower half, from their connection point with the chambers to their central section.

4. Digester according to Claim 1, **characterised by** the fact that two chambers are equipped with loading inlets on the lower half of the chamber and are connected, via this inlet and a system of tubes, to a storage tank for treatable waste.

5. Digester according to Claim 1, **characterised by** the fact that each chamber is connected, via a total extraction outlet and corresponding system of tubes, to a storage tank.

6. Digester according to any of the foregoing claims, **characterised by** the fact that all inlets, outlets and interconnecting pipes are fitted with shutoff valves or gates.

7. Digester according to Claim 1, **characterised by** the fact that the two propeller paddles have a circular-sector profile, a radius that is slightly lower than the internal radius of the module and they are positioned on opposite sides of the axis of rotation.

8. Digester according to Claim 1, **characterised by** the fact that it comprises three equidistant chambers positioned at the vertices of an equilateral triangle.

9. Digester according to Claim 1, **characterised by** the fact that each spherical chamber has an access gate on its lower half.

10. Digester according to Claims 2, 3 and 6, **characterised by** the fact that the shutoff valve on the pipes between the spherical modules are located in the central section of the smallest part of these pipes.

11. Digester according to Claim 1, **characterised by** the fact that it includes a continuous monitoring system for controlling transformation processes, biogas production and the characteristics of the output waste. It includes supervision, control and data-acquisition devices that provide information about the main parameters that characterise the process, for management and intervention purposes.

12. Digester according to Claim 1 or 10 **characterised by** the fact that it has a safety mechanism that limits the pressure of the gas produced by anaerobic fermentation in each chamber.

13. Digester according to Claim 1 or 6 **characterised by** the fact that the tubing from the discharge outlet for the waste broken down in each chamber runs on a downward slope and has shut-off valves at each end to stop air from getting into the digester.

14. Digester according to Claim 1 or 5 includes devices for storing recoverable products and sub-products: feeder tank for introducing new material into the system, gas holder for biogas and tanks for collecting the treated digestate from partial extraction, for use as compost, or for collecting material from total extraction.

15. Method of treating organic waste **characterised by** the fact that it involves the progressive and continuous degradation of organic waste through at least three equally sized and equidistant spherical modules positioned at the same height and connected in series; in each module, the waste is homogenised and treated through continuous mixing. New waste is selectively loaded into the chambers through loading inlets on two of the modules, while the treated digestate is selectively extracted from any of the three modules and the biogas is extracted simultaneously from all modules.

16. Method according to Claim 15 **characterised by** the fact that the extraction of treated digestate is selective, while the production of biogas is continuous.
